# EUROPEAN PATENT SPECIFICATION

(11) **EP 0 537 171 B1**
(45) Date of publication and mention of the grant of the patent: **18.09.2002**
(21) Application number: 91909416.9
(22) Date of filing: 10.05.1991
(51) Int. Cl.: C12N 15/12, C07K 14/00, C12P 21/08, C12Q 1/68, A61K 38/00, C07K 2/00

(54) **CD46 isoforms**
CD46 Isoformen
CD46 isoformes

(30) Priority: 11.05.1990 AU 13390
(43) Date of publication of application: 21.04.1993
(73) Proprietor: THE AUSTIN RESEARCH INSTITUTE, Heidelberg, VIC 3801 (AU)
(72) Inventor: PURCELL, Damian, Francis, John, South Gisborne, VIC 3437 (AU); RUSSELL, Sarah, May, North Fitzroy, VIC 3068 (AU); McKENZIE, Ian, Farquar, Campbell, Brunswick West, VIC 3055 (AU)
(74) Representative: Allam, Peter Clerk
(86) International application number: AU9100199
(87) International publication number: WO91018097

(56) References cited:
- WO-A-91/02002
- WO-A-91/05855
- EUROPEAN JOURNAL OF IMMUNOLOGY vol. 18, no. 8, 1988, VCH VERLAGSGESELLSCHAFT, DEUTSCHLAND pages 1288 - 1294 Seya, T. et al.; 'Distribution of membrane cofactor protein of complement on human peripheral blood cells. An altered form is found on granulocytes.'
- JOURNAL OF IMMUNOLOGY. vol. 138, no. 11, 1 June 1987, BALTIMORE US pages 3850 - 3855 BALLARD, LL ET AL.; 'A polymorphism of the complement regulatory protein MCP (Membrane Cofactor Protein or gp45-70).'
- BIOCHEMICAL JOURNAL vol. 264, no. 2, 1 December 1989, pages 581 - 588 SEAY, T. ;ATKINSON JP; 'Biochemical characterization of membrane cofactor protein of the complement system.'
- The Journal of Experimental Medicine, Volume 168, July 1988, pages 181-194, LUBLIN D. et al, "Molecular cloning and chromosomal localization of human membrane cofactor protein (MCP)", especially pages 184 and 188-189.
- The Journal of Immunology, Vol. 141, No. 11, December 1, 1988, pages 3923-3929, BALLARD L. et al., "Biochemical characterization of membrane cofactor protein of the complement system", see Abstract, and page 3927, column 1 para 2, page 3298 column 2.
- Immunology and Cell Biology, Vol. 67, No. 5, 1989, pages 279-289, PURCELL et al., "The Human non-lineage antigen CD46 (HULY-M5) and primate retroviral gp70 molecules share protein defined antigenic determinants", see specially pages 286-288.
- Immunogenetics, Vol. 31, 1990, pages 21-28, PURCELL et al., "Human non-lineage antigen, CD46, (HULY-M5): Purification and partial sequencing demonstrates structural homology with complement-regulating glycoproteins", see specially the Abstract and pages 27-28.
- CHEMICAL ABSTRACTS, Vol. 107, No. 9, issued August 31, 1987; BALLARD et al., "A polymorphism of the complement regulatory protein MCP (membrane cofactor protein or gp 45-70)", see page 536 Abstract No. 75801Y, Journal of Immunology 1987, 138 (11), pages 3850-5.
- CHEMICAL ABSTRACTS, Vol. 110, No. 17, issued April 24 1989; BORA, N.S. et al., "Structural gene for human membrane cofactor protein (MCP) of complement maps to within 100 Kb of the 3' end of the C3b/C4b receptor gene", see page 220, Abstract No. 149065X, Journal of Experimental Medicine 1989, 169(2), pp. 597-602.

## Description

This invention relates to isoforms of the protein CD46 as claimed in claim 1, a molecule that exists on the surface of almost all human cells to protect them from destruction by the normal immune processes. The invention also relates to nucleic acid encoding said CD46 isoforms, and vectors and cells containing such nucleic acid.

The human immune system operates on several levels of specificity and efficiency to identify and eliminate foreign organisms and substances without damaging or destroying normal (or autologous) cells. The complement proteins in serum form a relatively non-specific arm of immunity that plays a leading role in the initial detection, destruction and removal of foreign substances, and in triggering the specific arms of the immune response; formation of antibody and cell-mediated immunity and inflammation mediated responses. The complement proteins also play an important role in the later phases of an immune response by facilitating the phagocytosis of debris and the transport of immune-complexes and macromolecular debris to the liver or spleen for further processing and destruction. The central molecule in the complement system is C3b which aggregates in increasing amounts on foreign substances or organisms tagging them for removal. The complement precursor proteins are activated to form C3b in two ways: (i) by interacting with antibody bound to the foreign target (the classical pathway) or (ii) non-specifically, by progressive and rapidly increasing accumulation on foreign surfaces (the alternative pathway). In order to control this process of complement activation and protect normal cells and tissues from indiscriminate destruction, a family of cell-surface molecules have evolved that interact with C3b molecules; these are:
(i) CD46 (also known as membrane cofactor protein [MCP]) which exists on all normal human cells, except red blood cells, and binds to C3b and activates molecules that cleave C3b into inactive fragments before it can accumulate sufficiently to destroy the cell.
(ii) CD55 (also known as decay accelerating factor [DAF]) which exists on all cells, including red blood cells, and prevents C3b from reacting with other complement components preventing the destructive processes, however, unlike CD46, this does not destroy C3b.
(iii) CD35 (or complement receptor 1 [CR1]) which exists on a select group of leukocytes and causes the degradation of C3b molecules adhering to neighbouring cells.

CD46 and CD55 (MCP and DAF) are the predominant molecules protecting normal autologous tissues from complement-mediated destruction. The absence of these C3b receptors on foreign cells or substances labels them as foreign and promotes their destruction.

Leukaemia cells and other malignant tumour cells express a greatly elevated level of CD46 at their surface which is biochemically and structurally different (but still related to CD46) on the corresponding non-malignant cell. The surface molecules of primate retroviruses that cause leukaemia and AIDS, the gibbon ape leukaemia (GaLV) and Mason-Pfizer monkey (MPMV) viruses, have a structural - protein component that resembles CD46 as demonstrated by the binding of the monoclonal antibody E4.3 (the benchmark reagent defining CD46) to these viral molecules. The existence of these altered (or mimicked) versions of CD46 in high levels on tumour cells and viruses is likely to enhance their evasion of complement-mediated immunity.

CD46 also appears to play an important role in human reproduction as human spermatozoa express high levels of a novel form of CD46 at the surface after the acrosome reaction. This occurs prior to fusion of sperm with the oocyte. CD46 molecules are also secreted extracellularly from the spermatozoa after the acrosome reaction. These findings suggest that CD46 plays an important role in the process of fertilization and recombinant (synthetic) CD46 may facilitate the process of fertilization.

The layer of syncytiotrophoblast cells in the placenta, the interface between the mother and the antigenically different fetus, expresses higher levels of CD46 than any other tissue. CD46 is the same as the TLX molecule identified by reproductive immunologists as a crucial molecule protecting the non-identical fetus tissues from immune-rejection by the mother. It is possible that in addition to the ability to regulate the activation of complement, CD46 dampens the specific immune responses, even the strong response to genetically different cells.

CD46 is thus an important molecule regulating the immune response and may have a role in preventing complement-mediated tissue damage, to enhance immunity to tumours and viruses, to control the process of fertilization, to prevent recurrent spontaneous abortion of the fetus during pregnancy, and to facilitate the engrafting of transplanted tissues.

The CD46 polypeptide has been identified by gel electrophoresis as a heterogenous group of glycoproteins resolving in two diffuse bands known as the α and β chains, having molecular weight of 56,000 and 60,000 daltons respectively, which are not linked by disulphide bonds. The α and β chains of CD46 have been shown to have the same N-terminal amino acid sequence. It has previously been thought that the differences between the α and β chain may be due to glycosylation differences.

In Lublin *et al.*, (1988, J.Exp.Med., vol 168, pp 181-194), the molecular cloning of an MCP cDNA is described.

We describe the surprising discovery that there are at least fourteen RNA splice variants encoding CD46 molecules. As will be hereinafter described, the various CD46 splice variants have a conserved N-terminus but different protein sequences at the carboxyl-terminal end.

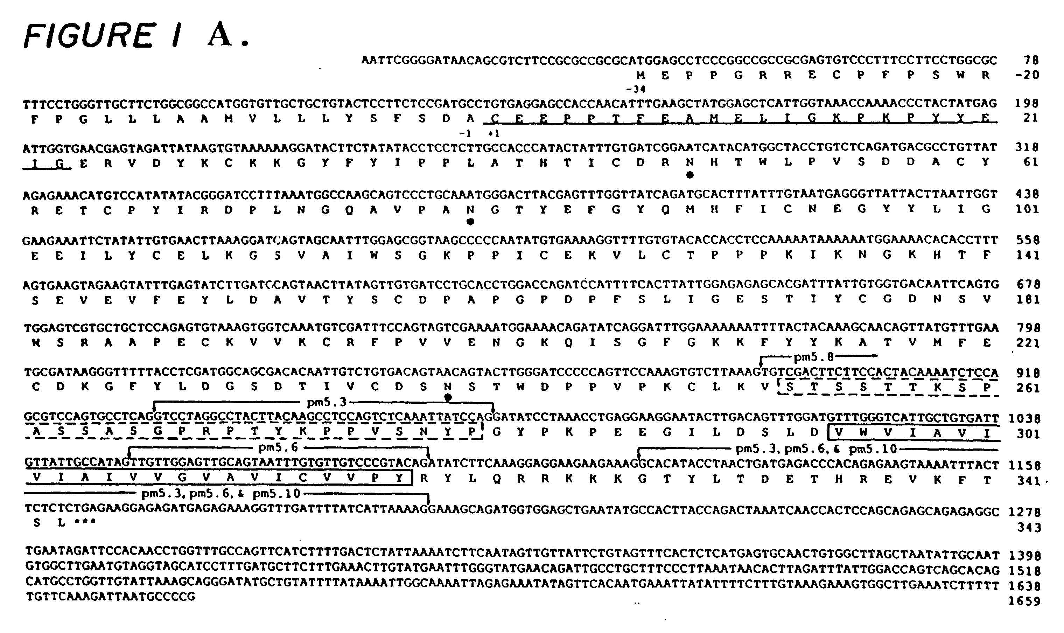

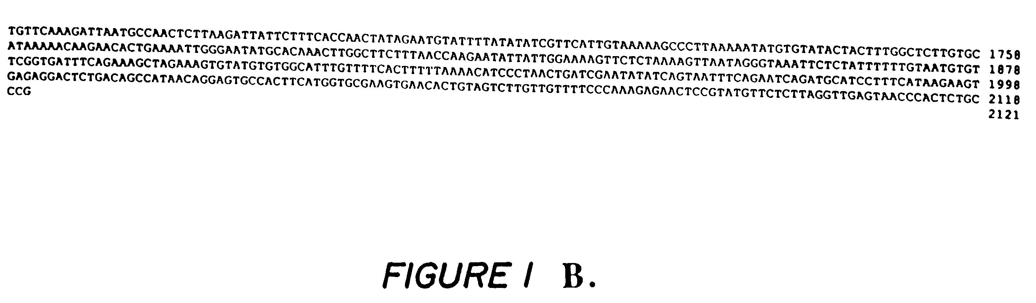

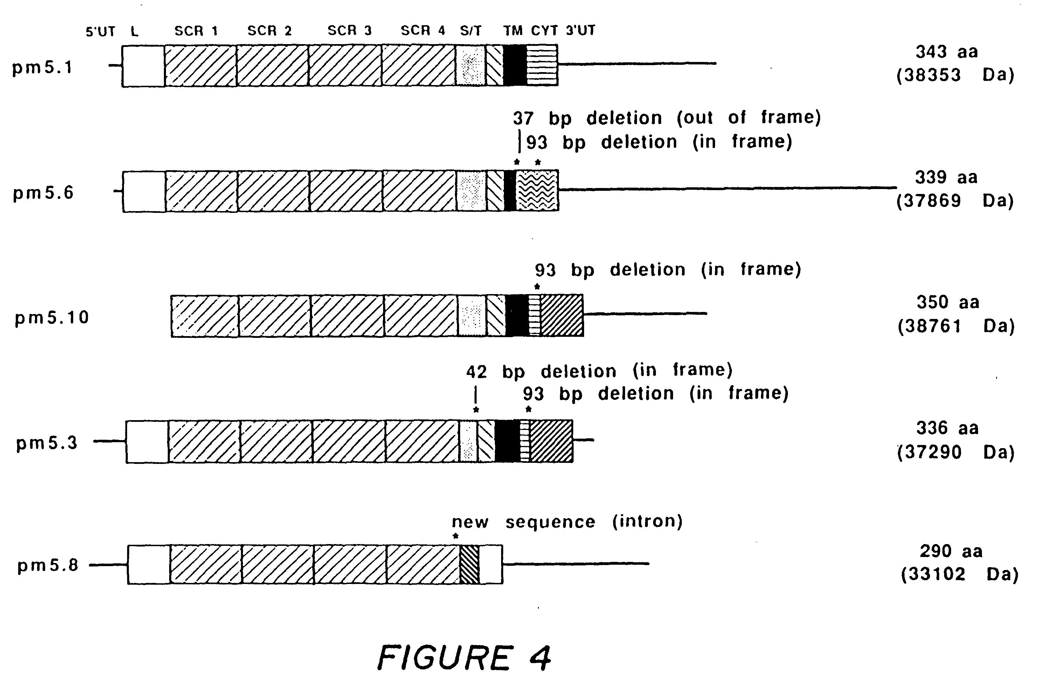

Broadly speaking, the invention encompasses recombinant nucleic acids encoding particular CD46 isoforms which are shown in Fig.4 in conjunction with Fig 1A.

We describe nucleic acid comprising a splice variant of the structural gene encoding the CD46 family of proteins with the proviso that the splice variant does not consist of exons 1 to 6, 8 to 12 and 14 (as herein defined) of the structural gene. This excludes the published sequence of MCP appearing in Lublin et al. (1988) J. Exp. Med., 168, 181-184.

In accordance with a first aspect of this invention we provide a recombinant nucleic acid encoding: (a) a CD46 isoform defined by cDNA clone pm5.1, as shown in Figure 4 in conjunction with Figure 1A; (b) a CD46 isoform defined by cDNA clone pm5.3, as shown in Figure 4 in conjunction with Figure 1A; (c) a CD46 isoform defined by cDNA clone pm5.6, as shown in Figure 4 in conjunction with Figure 1A; or (d) a CD46 isoform defined by cDNA clone pm5.8, as shown in Figure 4 in conjunction with Figure 1A.

According to a second, and alternative, aspect of the invention, we provide a probe comprising a nucleic acid according to the first aspect of the invention and a detectable marker linked thereto.

There is provided, in accordance with a third, and alternative, aspect of the invention, a eukaryotic or prokaryotic transfer or expression vector comprising a nucleic acid sequence according to the first aspect of the invention and a promoter sequence therefor.

There is provided, according to fourth and fifth alternative aspects of the invention, a host cell compatible with and incorporating a vector according to the third aspect of the invention, whereby the host cell is capable of expressing the CD46 isoform; and a host cell having a nucleic acid according to the first aspect of the invention operatively integrated within its genome, whereby the cell is capable of expressing the CD46 isoform.

According to a sixth alternative aspect of the invention, we provide a CD46 isoform produced by a cell as defined in the fourth or fifth aspects of the invention.

We provide, according to a seventh, alternative aspect of the invention, a pharmaceutical composition comprising a CD46 isoform as defined in the sixth aspect of the invention in conjunction with a pharmaceutically acceptable carrier or diluent.

In accordance with further, alternative, aspects of the invention, we provide for the use of a CD46 isoform as defined in the sixth aspect of the invention in the manufacture of a medicament or diagnostic reagent; and the use of a nucleic acid as defined in the first aspect of the invention in the manufacture of a probe or antisense therapeutic agent.

We further provide, according to a further, alternative, aspect of the invention, a CD46 isoform as defined in the sixth aspect of the invention for use in a method of inhibiting complement activation or inflammation mediated immunoresponse in a mammal.

In particular, we provide an antisense oligonucleotide which is complementary to at least a substantial portion of exon 7 of a recombinant nucleic acid as defined in the first aspect of the invention, for use in a method of inhibiting leukaemic cells, the method comprising administering antisense oligonucleotide to an animal, the antisense oligonucleotide serving to diminish the expression of CD46 by the leukaemic cells thereby rendering the cells vulnerable to complement.

We further provide, in accordance with yet another aspect of this invention, a nucleic acid probe as defined in the second aspect of the invention for use in a method of diagnosing the likelihood of spontaneous abortion in a mammal, the method comprising applying the probe to tissue or fluid of the mammal or its foetus.

In accordance with yet further aspects of the invention, we provide a CD46 isoform as defined in the sixth aspect of the invention for use in a method of maintaining pregnancy in a habitually aborting mammal, the method comprising the administration of the CD46 isoform to the mammal to thereby mop up complement; and a CD46 isoform as defined in the sixth aspect of the invention for use in a method of preventing inhibition of mammalian sperm function, the method comprising the administration of an effective amount of the CD46 isoform to the sperm or its environment.

The exon structure and assignment of the sequence of Figure 1 is discussed in more detail in Examples 4 to 7 which follow. Briefly, the sequence depicted in Figure 1 corresponds to a leader peptide encoded by a single exon (exon 1), four extracellular short consensus repeat (SCR) domains (characteristic of the family of complement regulating proteins as described by Lublin et al. (1988) J. Exp. Med. 168, 181-194) encoded by exons 2-6, an extracellular region rich in Ser Thr and Pro residues (STP) encoded by exons 7 to 9, a short region of unknown significance (exon 10), a hydrophobic membrane spanning region (TM) encoded by exons 11 and 12 and a short cytoplasmic tail (CYT) encoded either by exon 13 or exon 14, each of which contain a stop codon.

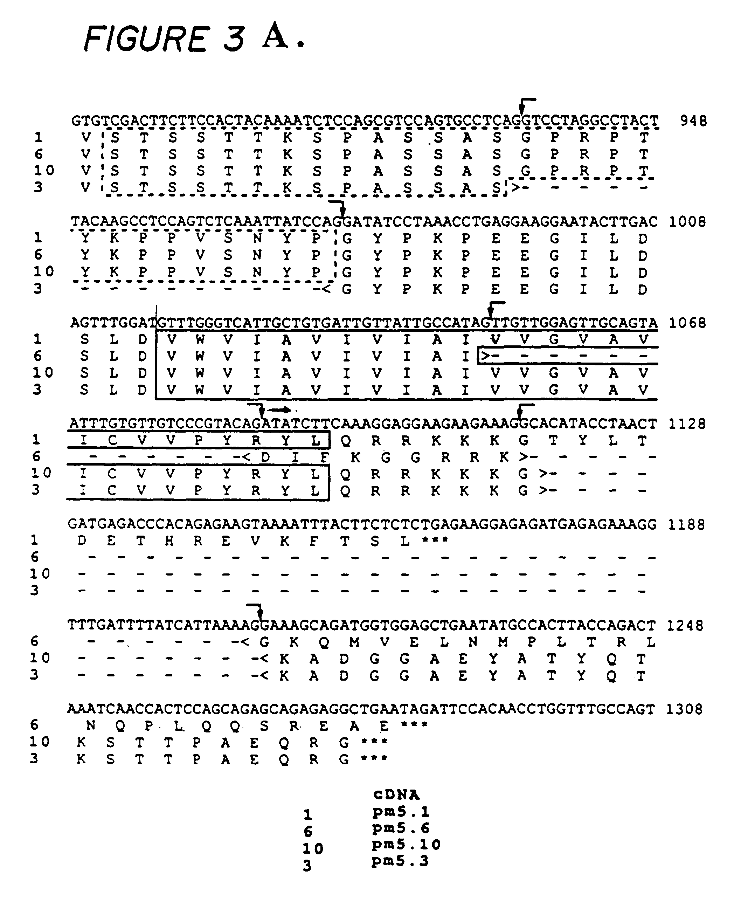

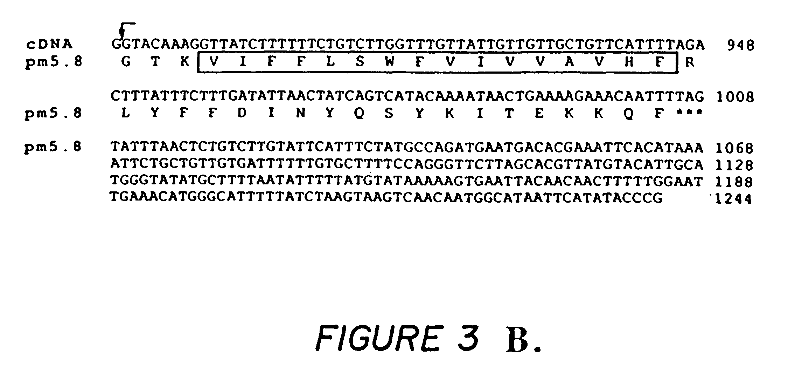

Some of the nucleic acids described here correspond to spliced variants of mRNA encoded by the structural gene-encoding CD46. The term "nucleic acid" as used herein generally refers to DNA, such as cDNA. However, this term also embraces mRNA. Nucleic acid encoding CD46 isoforms specifically excludes the known nucleotide sequence encoding the protein known as MCP, which we have now found corresponds to nucleic acid encoding a specific CD46 variant referred to herein as pm5.10 (see Figure 3).

Antisense oligonucleotides directed selectively to exon 7 of the CDNA shown in Fig.4 in conjunction with Fig. 1A could inhibit the production not only of this exon but of the whole molecule. Such cells would then not express CD46 and could be susceptible to lysis by complement and lead to selective destruction of tumours.

Nucleic acid of the invention are the following:
PM5.1 - a nucleic acid encoding a protein of about 377 amino acids, and being characterised by having:
   (i) a first region encoding four contiguous substantially homologous units of approximately 60 amino acids and referred to as SCR (short consensus repeats);
   (ii) a second region encoding a serine-threonine rich sequence of approximately 45 amino acids;
   (iii) a third region encoding a transmembrane domain of approximately 23 amino acids; and
   (iv) a fourth region encoding a 26 amino acid cytoplasmic domain containing predominantly charged amino acids.

The complete nucleotide sequence of PM5.1 is given in Figure 1.

PM5.3 -- A nucleic acid corresponding to PM5.1 with the exception that:
(i) a 42 base pair fragment encoding 14 amino acids is deleted from the second, serine-threonine rich, region; and
(ii) a 93 base pair fragment is deleted from the fourth region encoding the cytoplasmic tail.

Due to the loss of a termination codon this encodes a variant cytoplasmic tail having the first seven amino acids of the PM5.1 cytoplasmic tail, and then a further 23 amino acids arising from 3' untranslated sequences of PM5.1.

The variant C-terminus of PM5.3 is shown in Figure 3.

PM5.6 -- A nucleic acid corresponding to PM5.1 with the exception that as for PM5.3, a 93 base pair fragment is deleted from the fourth region encoding the cytoplasmic tail. Additionally, a 37 base pair fragment is deleted from the transmembrane region (encoding the last 12 amino acids thereof) due to the use of a cryptic splice acceptor signal. The absence of this fragment changes the reading frame of the carboxy terminal end of CD46 to encode a peptide having 34 amino acids distinct from those encoded by other CD46 nucleic acids.

The C-terminal sequence of PM5.6 is shown in Figure 3.

PM5.8 -- A nucleic acid corresponding in part to 5.1 with the exception that those regions after the first region encoding the contiguous SCR regions are replaced with an intron sequence encoding 16 hydrophobic amino acids.

The C-terminal nucleotide sequence of PM5.8 is also given in Figure 3.

We further describe a transfer or expression vector which encodes a nucleic acid in accordance with this invention. Transfer or expression vectors include plasmid and viral DNAs, or viral RNA, but are not limited thereto. Any vehicle capable of replication in a host cell, whether prokaryotic or eukaryotic, is within the scope of this invention. Generally, but without limitation, a transfer or expression vector will include an origin of replication, promoter, selective marker (such as antibiotic resistance), and a nucleic acid sequence encoding CD46 as herein described, under the control of a promoter.

We also describe a cell containing a transfer or expression vector and CD46 produced by such a cell. Suitable cells may be prokaryotic or eukaryotic. The nature of the cell is generally unimportant as long as the transfer vector is capable of replication and desirably expressing therein. Eukaryotic cells are generally used when CD46 is to be post-translationally modified by the addition of carbohydrate, etc. Prokaryotic cells, such as bacterial strains, are preferred when post translational modification is not desired or is considered unimportant. For instance, vectors pAV009/A⁺, pKC3 and pEE6/hCMV/GSCDM8 and other vectors are appropriate for expression in Cos and Chinese hamster ovary cells. pGEX-3X is useful for prokaryotic expression in E. coli. We have observed that sequences encoding CD46 isoforms contain two possible translational start sites (rather than the single site originally reported). Although the 5'-most ATG is preferred this site does not appear to be in the optimal sequence context in the CD46 family of proteins. Accordingly to improve expression PCR can be used to amend the 5' ATG to the consensus sequence or the 5' ATG removed to allow more effective translation from the distal ATG. Similarly, as untranslated regions of some RNAs may effect stability of the RNA and translation efficiency, removal of 3' and 5' untranslated regions, for instance by PCR may be desirable.

Vectors containing CD46 nucleic acid sequences may be introduced into cells by well known methods such as electroporation, conjugation, calcium phosphate precipitation, transformation of competent cells (such as bacterial cells after treatment with CaCl₂) and the like. Cells may have nucleic acid encoding CD46 as described herein integrated into the host chromosome.

CD46 produced by host cells may be isolated by protein purification techniques such as chromatography on size-exclusion or ion exchange matrices, HPLC, protein precipitation, affinity chromatography (using, for example, antibodies directed against CD46) and like techniques which are well known in the art. We further describe essentially pure CD46 isoforms translated from a nucleic acid.

The CD46 protein sequence may be subject to side chain modifications such as:
Modifications of amino groups including
   - Reductive alkylation by Schiff's base formation by an aldehyde followed by reduction with NaBH₄.
   - Amidination with methylacetimidate.
   - Acylation with acetic anhydride.
   - Carbamoylation of amino groups with cyanate.
   - Trinitrobenzylation of amino groups with 2,4,6, trinitrobenzene sulphonic acid (TNBS).
   - Acylation of amino groups with succinic anhydride and tetrahydrophthalic anhydride.
   - Pyridoxylation of lysine with pyridoxal-5'-phosphate followed by reduction with NaBH₄.
Modification of the guanidino group of Arg by the formation of heterocyclic condensation products with reagents such as 2,3 butanedione, phenylglyoxal and glyoxal.
Modification of carboxyl groups by carbodiimide activation via O-acylisourea formation followed by subsequent derivitisation e.g. to a corresponding amide.
Modification of sulphydryl groups including
   - Carboxymethylation with iodoacetic acid or iodoacetamide.
   - Performic acid oxidation to cysteic acid.
   - Formation of a mixed disulphide with 5,5' dithiobis(2-nitrobenzoic acid) (DTNB).
   - Reaction with maleimide, maleic anhydride or other substituted maleimide. Formation of mercurial derivatives using 4-chloromercuribenzoate, 4-chloromercuriphenylsulphinic acid, phenylmercury chloride, 2-chloromercuri-4-nitrophenol and other mercurials.
   - Carbamoylation with cyanate at alkaline pH.
Modification of imidazole ring of histidine including
   - Alkylation with iodoacetic acid derivatives and
   - N-carbethoxylation with diethylpyrocarbonate.
Modification of tryptophan residues including
   - Oxidation of N-bromosuccinimide.
   - Alkylation of the indole ring with 2-hydroxy-5-nitrobenzyl bromide or sulphenyl halides.
Modification of the tyrosine residues
   - Nitration with tetranitromethane to form 3-nitrotyrosine derivatives.
Modifications as set out above may be produced according to standard chemical reactions well known in the art.

CD46 as herein described may be provided in the form of therapeutic compositions in association with one or more pharmaceutically acceptable carriers or excipients. Examples of carriers or excipients which may be utilized in this invention are provided in Remington's Pharmaceutical Sciences 16th Ed, 1980, Mack Publishing Company, Osoll et al, which is incorporated herein by reference. By way of example only, CD46 according to the invention may be in association with saline, albumin, water, dextrose or the like. CD46 isoforms may also be used in the manufacture of a medicament. The CD46 isoforms in accordance with this may be utilised to prevent complement-mediated or inflammation mediated tissue damage, to enhance immunity to tumours and viruses, to control the process of fertilization, to prevent recurrent spontaneous abortion of the fetus during pregnancy and to facilitate the engrafting of transplanted tissue.

For example inhibition reactions of complement activation in the classical pathway are initiated by antibody binding to antigen and examples are blood transfusion reactions, glomerulonephritis, Goodpasture's syndrome, systemic lupus erythematosis, rheumatoid arthritis, graft rejection and other diseases where antibody interact with antigen. In addition, certain aspects of cellular immunity are mediated by the complement pathway such as complement receptor on macrophages, polymorphs and other cells binding to antigen-antibody-complement complexes, CD46 isoforms could inhibit such reactions. In addition, CD46 isoforms would be expected to inhibit the alternative pathway activated by inflammatory mediators, bacteria and other substances. Such activation occurs in most inflammatory conditions not necessarily of antibody, i.e. immunological, origin. For example, inflammation which occurs in myocardial infarction, hepatitis, pneumonia, gastroenteritis; indeed inflammation of any organ. Certain aspects of the inflammation are beneficial, i.e. to eradicate bacteria, viruses and the like. However, certain aspects of inflammation are non-specific and cause tissue damage. Soluble CD46 according to the invention could inhibit such non-specific reactions.

An example of the diagnostic and therapeutic utility of the CD46 isoform nucleic acids according to claim 1 or proteins is described above with regard to exon 7 expression in leukaemic cells. It is also, not unlikely that the presence or absence of exons found in certain sequences are responsible for retention/rejection of the fetus in pregnancy. From this, diagnostic tests, for instance nucleic acid probing of maternal or fetal tissue or fluids could be performed to determine which individuals are likely to abort. Administration of a soluble CD46 encoded by the clones of claim 1 may serve to mop up surrounding complement to maintain a pregnancy in an habitually aborting mother. antisense oligonucleotides as described here will be useful to cause or prevent the expression of these exons and thus overcome the problem of habitual abortion.

A further utility of nucleic acids and CD46 proteins lies in the field of male infertility and encouraging fertilization. It has been observed above that certain of the CD46 family are uniquely expressed on sperm cells, presumably to inhibit complement activated lysis. Individuals with azospermia may be diagnosed with antibodies or probes in accordance with the invention, directed to the unique structure of the sperm CD46. As mentioned above soluble CD46 promises to be useful in mopping up complement to facilitate survival of otherwise vulnerable sperm or to otherwise prevent inhibition of sperm function i.e. migration, penetration, motility or fertilization. It is also likely that CD46 on the sperm is part of a receptor complex responsible for sperm binding to oocytes prior to fertilization. It will be apparent that nucleic acids or CD46 proteins will be useful in fertility control.

Nucleic acids and CD46 proteins as claimed in claim 1 also find utility in transplantation. In the rejection of allografts, i.e. from one human to another, T cells are involved and establish inflammatory mechanisms. The CD46 isoforms of claim 1 could inhibit such non-specific inflammatory reactions occurring after the specific event. In addition, allo-antibodies are involved either in hyperacute rejection or in the late phase of rejection and these act using complement. Said CD46 may also inhibit this procedure. With regard to xenotransplantation, i.e. form one species to another, it is possible that CD46 could have several actions. In the first soluble material could inhibit the action of complement which is involved in rejection. In the second, transgenic non-human animals could be made expressing the claimed CD46 on the cell surface could be used for such a procedure. In this setting, one could envisage such species as pigs, sheep or other non-human species expressing human CD46 being used for transplantation experiments. The human CD46 in these organs would break down complement as it was deposited and make the tissues resistance to complement mediated lysis. It is possible that such xenografts could become universal donors for transplants to humans. The pm5.1 clone, containing the CYT 1 tail, is the most likely candidate for this function as it has been shown to have increased cell surface expression compared to clones with the CYT 2 tail as for instance expressed in MCP or pm5.3, pm5.10.

Specific embodiments will now be described by way of example only, with reference to the following non-limiting Figures and Examples.

### FIGURES:

**Figure 1A** shows the complete nucleotide sequence and predicted amino acid sequence of the pm5.1 cDNA clone of CD46. The NH₂-terminal amino acid sequence obtained from purified CD46 glycoprotein is underlined; potential addition sites for N-linked carbohydrate are marked with an asterisk; the region boxed with a hashed line is rich in Ser/Thr residues - potential O-linked glycosylation sites; the region boxed with a solid line is the hydrophobic transmembrane domain. The regions within the labelled arrows are the segments of DNA deleted by the other clones, all of the sequence beyond the arrow labelled pm5.8 has been exchanged for new sequence in the pm5.1 clone.

**Figure 1B** shows the sequence of additional nucleotides in the 3' untranslated region of the pm5.6 clone, which is longer than the pm5.1 sequence shown in Figure 1A. The numbering corresponds to the position relative to the pm5.1 clone and does not take account of the 130 bp deleted from the coding sequence of pm5.6.

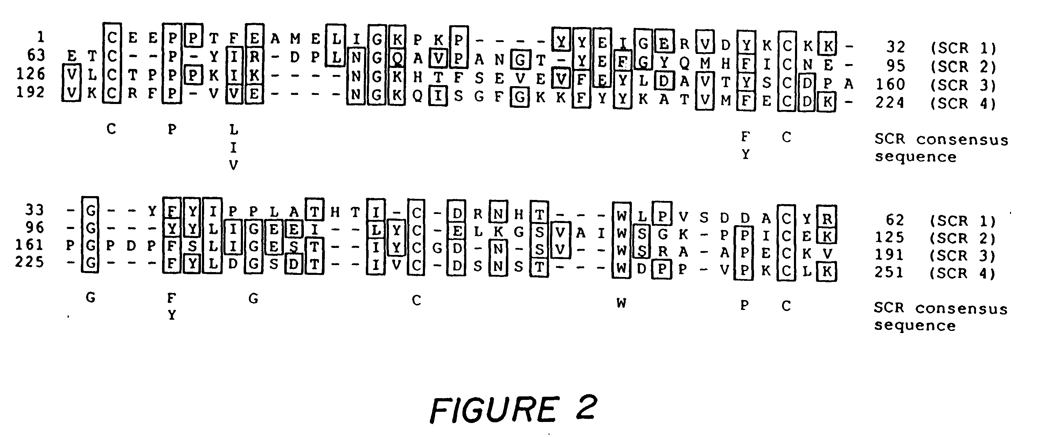

**Figure 2** shows the alignment of the NH₂-terminal 251 amino acids of the mature CD46 protein showing the four domains of internal homology, indicated by the boxes. Conservative substitutions are shaded. Spaces (-) have been introduced to maximise alignment. The alignment of these internally conserved residues with the consensus sequence of the 60- amino acid short consensus repeats of the complement regulatory proteins is shown at the bottom.

**Figure 3** depicts the alternate carboxyl-terminal protein sequences deduced from the nucleotide sequences of the different cDNA clones for CD46 commencing from amino acid 252. The amino acid sequences encoded by the pm5.1, pm5.6, pm5.10, and pm5.3 clones are shown in (A) the regions of DNA deleted in these clone is denoted by the arrows above the pm5.1 DNA sequence and the dashes (-) in the protein sequence. The DNA and amino acid sequence of pm5.8 that differs to that of the other clones is shown in (B) but is numbered relative to the pm5.1 clone. The regions of hydrophobic amino acids are shown with a solid box and regions rich in Ser and Thr residues are shown with a hatched box.

**Figure 4** shows a summary of the structure of the five alternate cDNA clones for CD46 showing the positions where differences in sequence occur relative to the pm5.1 clone. The location of the 5' untranslated (5' UT), leader peptide (L), short consensus repeat (SCR), serine/threonine rich (S/T), hydrophobic transmembrane (TM), cytoplasmic tail (CYT), and 3' untranslated (3' UT) regions are labelled above the pm5.1 clone. The number of amino acids and calculated molecular weight of each version of CD46 potentially encoded by these is shown at the right.

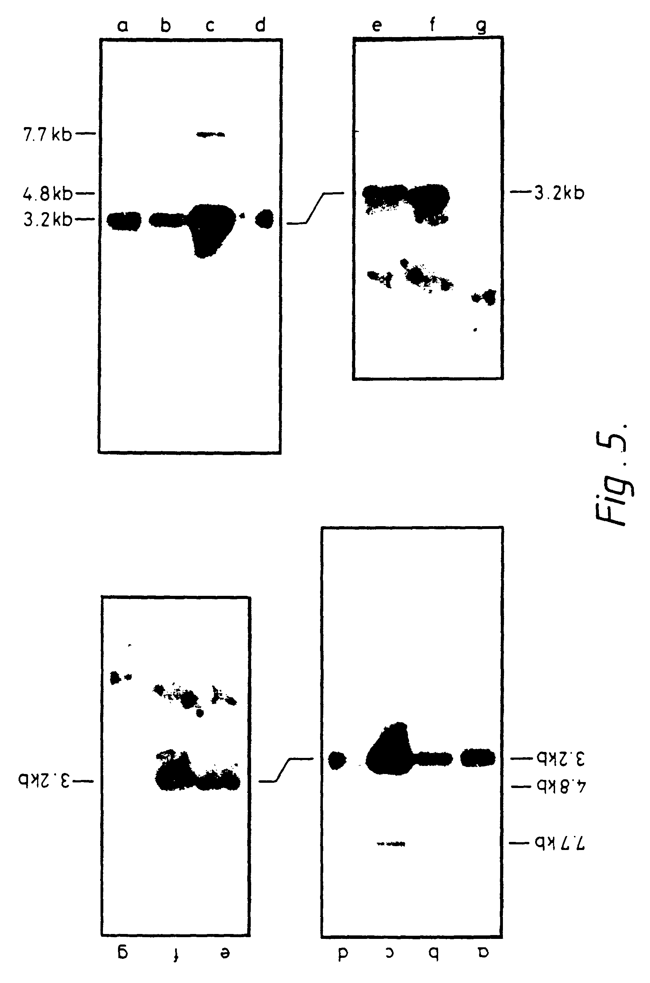

**Figure 5** is a northern blot analysis of 2µg of poly(A)⁺ RNA separated under denaturing conditions by electrophoresis on 1.2% agarose gels containing formaldehyde, transferred to Nylon membrane and hybridized with ³²P-labelled CD46 cDNA from the pm5.1 clone. The autoradiograph shows RNA from human spleen tissue from patients with non-Hodgkins lymphoma (lane a), polycythemia vera (lane b), hairy cell leukaemia (lane c), physical trauma (normal) (lane g) and from the human T-lymphoblastoid cell line PEER (lane c), the myelomonocytic cell line U937 (lane f), and the gibbon ape T-lymphoblastoid cell-line UCD-144-MLA (lane d). Each sample had a predominant band of 3.2kb and the spleen tissue infiltrated with hairy cell leukaemia cell had two minor bands at 4.8kb and 7.7kb.

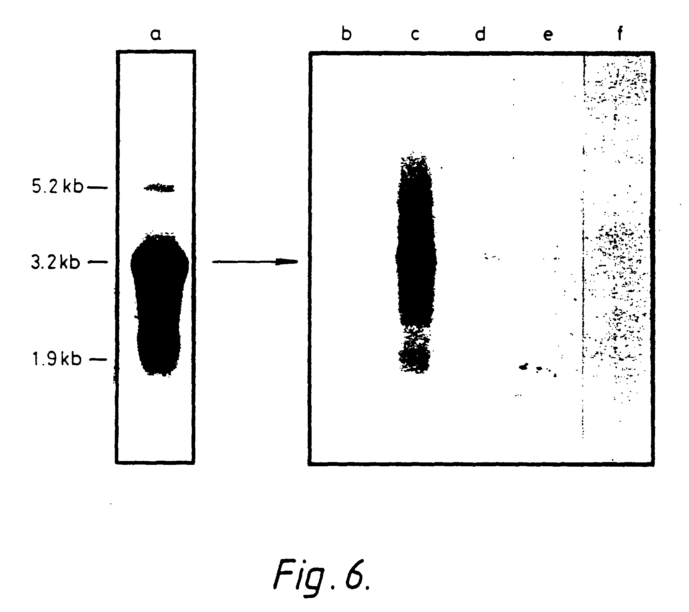

**Figure 6** is a northern blot analysis of 2µg of poly(A)⁺ from human placenta separated under denaturing conditions by electrophoresis on 1.2% agarose gels containing formaldehyde, transferred to strips of Nylon membrane that were hybridized with either ³²P-labelled CD46 cDNA from the pm5.1 clone (lane a), or the oligonucleotides; On63 which spans the site of the 93bp deleted from the pm5.1 sequence and therefore specific for RNA corresponding to the pm5.3, pm5.6 and pm5.10 clones (lane b), On46 which is within this 93bp segment of pm5.1 and therefore specific for RNA corresponding to the pm5.1 clone (lane c), On35 which spans the site of the 42bp segment deleted by the pm5.3 clone and therefore specific for RNA corresponding to the pm5.3 clone (lane d), On44 which is within the 42bp segment deleted by the pm5.3 clone and therefore specific for RNA corresponding to the pm5.3 clone (lane c), or the On68 which is within the new (intron) sequence of the pm5.8 clone (lane f). Details of these oligonucleotides are shown in Table 2.

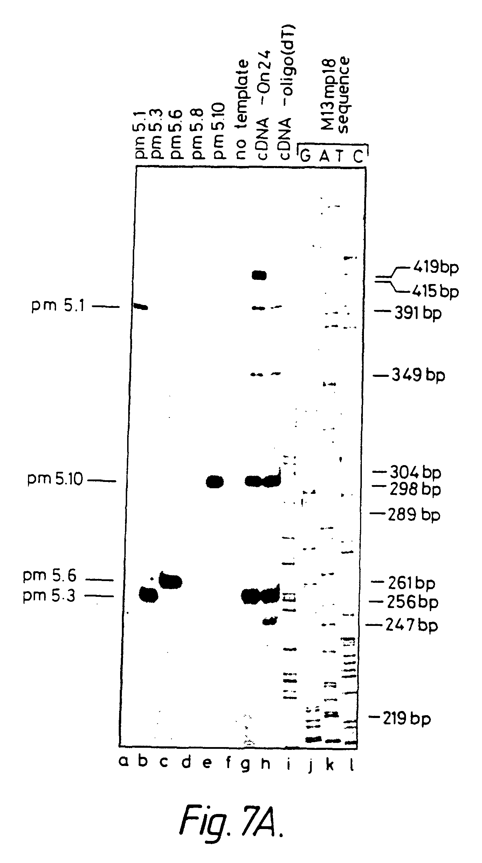

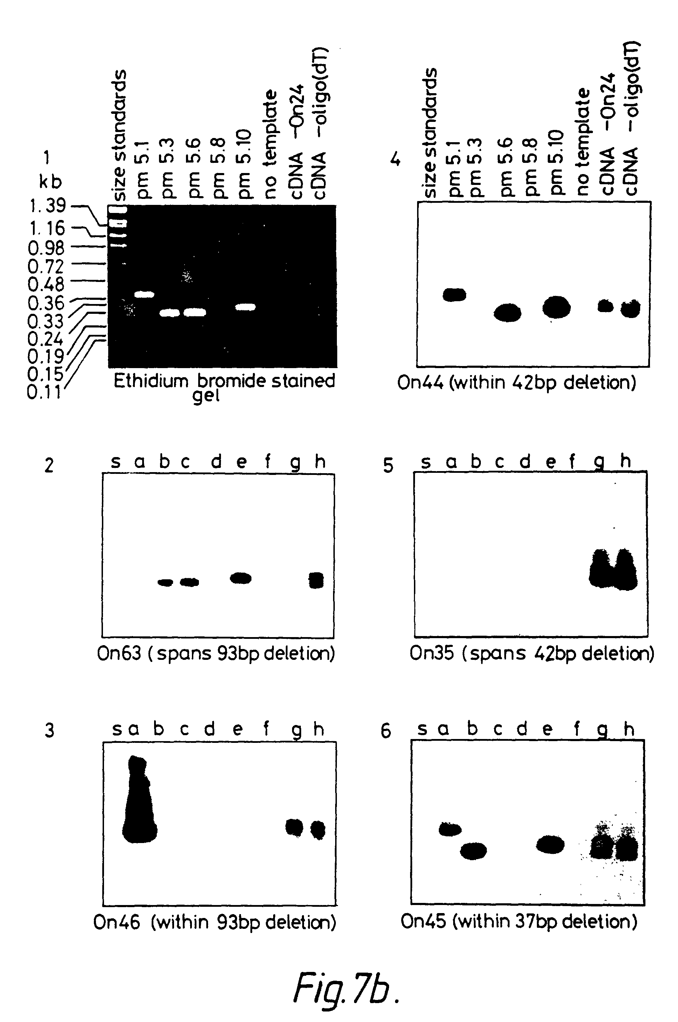

**Figure 7** Expression of CD46 transcripts in human placenta. The variable region of CD46 was amplified by PCR using primers On37 and On24. Templates for the PCR reaction were the clones pm5.1, pm5.3, pm5.6, pm5.8 and pm5.10 (lanes a to e respectively), no template (lane f), and cDNA produced from placental poly (A)+ cytoplasmic RNA primed with On24 (lane g) and oligo(dT) (lane h). A. ³²P-labelled samples were electrophoresed on a 6% acrylamide gel containing urea with the M13mp18 sequence ladder as a size marker. Sizes of the PCR products are indicated on the right and the position of the PCR products corresponding to the clones are arrowed on the left. B. Unlabelled products were electrophoresed on a 1.5% agarose gel (Panel 1), transferred onto nylon and probed with oligonucleotides specific for different CD46 transcripts (Panels 2 to 6). Details of the oligonucleotides are shown in Table 2.

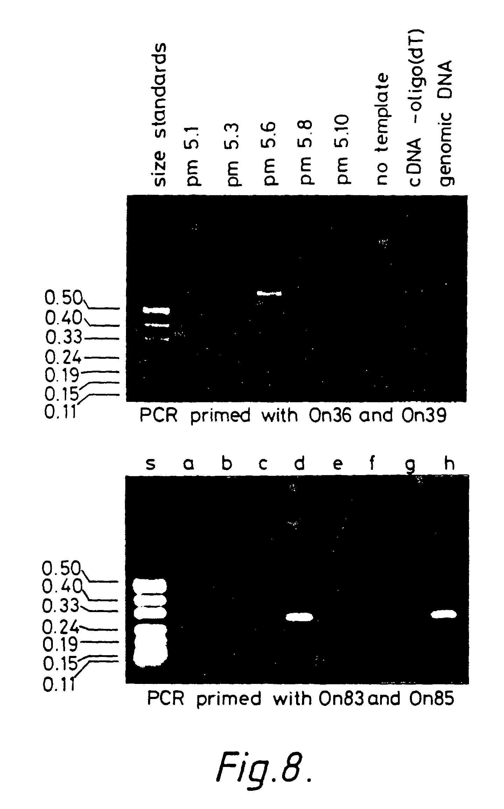

**Figure 8** Expression of pm5.6 and pm5.8 type transcripts in human placenta. The five clones (lanes a-e), placental cDNA from cytoplasmic RNA primed with oligo(dT) (1 lane g) or random hexamers (2 lane g) and placental genomic DNA (lanes h) were amplified with paris of primers including: (1) an oligonucleotide spanning the pm5.6 deletion (On36 and On39), or (2) on oligonucleotide in the new sequence of pm5.8 (On83 and On85), and electrophoresed on 1.5% agarose gels with Hpa 11 digested PUC19 DNA as a size marker (lanes s). Reaction mixes containing no template DNA were included as a control.

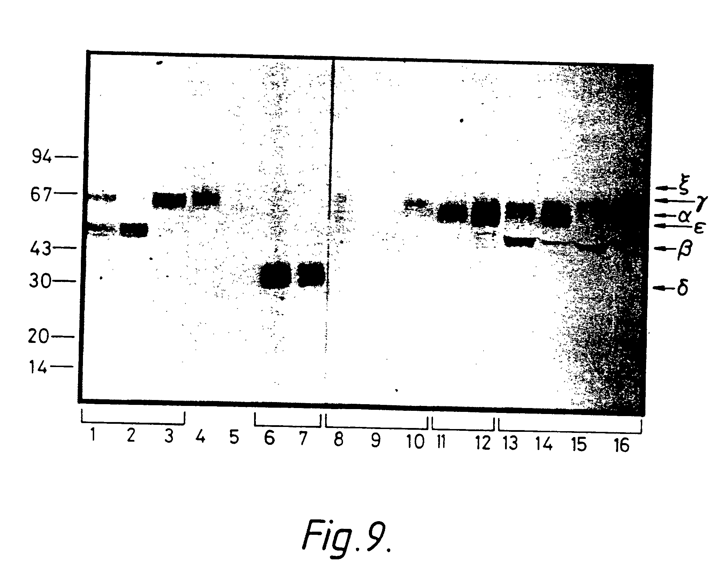

**Figure 9** Distribution of CD46 isoforms in various tissues. Western blot probed with E4.3 showing distribution of CD46 isoforms in lymphocytes (lanes 1 - 3), granulocytes (lane 4) and EBV-transformed B cells (lane 5), spermatozoa (lanes 6 and 7), leukemic cells (lanes 8 - 10), normal and malignant colon tissue from a patient with colon carcinoma (lanes 11 and 12 respectively), and full term placentae (lanes 13 - 16). Samples in lanes 3 - 6 are from the same donor as are samples in lanes 1 and 7. Relative molecular mass in kDa is shown at left and Greek letters at right indicate positions of isoforms.

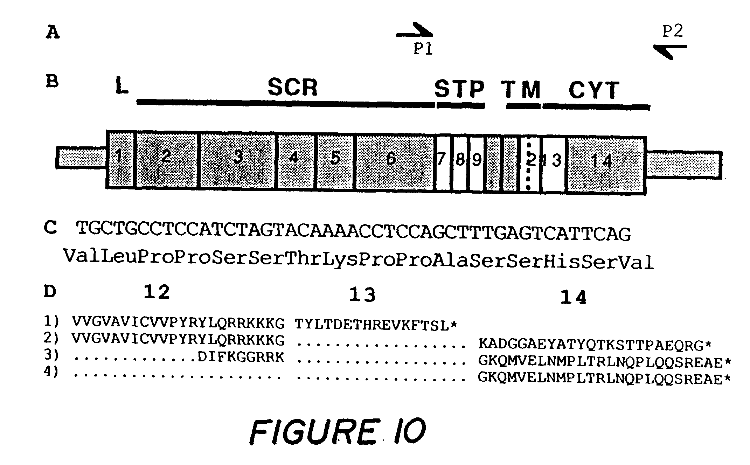

**Figure 10A** Position of primers used to amplify the region of alternative splicing. Sequence of P1:- 5'-GGCAGCGACACAATTGTC-3' and P2:- 5'-CAGCCTCTCTGCTCTGCTG-3'.

**Figure 10B** Exon organization of the CD46 gene; exons numbers are shown in boxes and functional domains of the derived amino acid sequence is shown above. Shaded boxes indicate constitutively spliced exons and white boxes indicate exons which are alternatively spliced. Dashed line indicates cryptic splice acceptor site.

**Figure 10C** Nucleotide and derived amino acid sequence of exon 7.

**Figure 10D** Derived amino acid sequence of the COOH-terminal region of the CD46 isoforms encoded by the differential splicing of exons 12 and 13. Spaces in amino acid sequence indicate deletions of the exons shown above and * indicates stop codons.

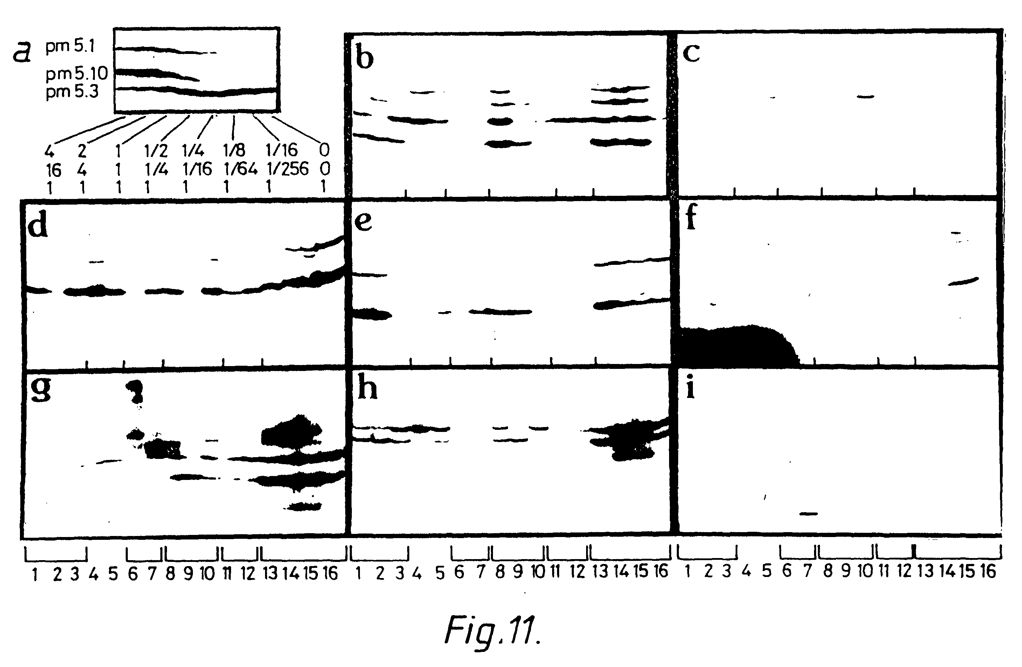

**Figure 11A** Amplification of CD46 cDNA clones using PCR. CD46 cDNA clones, pm5.1, pm5.3 and pm5.10 were amplified together in the proportions indicated below each track. α[³²P]dCTP was incorporated into the PCR products and the samples were electrophoresed and autoradiographed.

**Figure 11B** Electrophoresis of radiolabelled, amplified cDNA from lymphocytes (lanes 1 - 3), granulocytes (lane 4) and EBV-transformed B cells (lane 5), spermatozoa (lanes 6 and 7), leukemic cells (lanes 8 - 10), normal and malignant colon tissue from a patient with colon carcinoma (lanes 11 and 12 respectively), and full term placentae (lanes 13 - 16). samples 3-6 are from the same donor as are samples 1 and 7.

**Figures 11C-11I** Tissue distribution of individual exon combinations. Unlabelled, amplified cDNA from the same samples as 11B were electrophoresed, electroblotted and probed with oligonucleotides specific for different exons or exon combinations (see Figure 12 for locations of probes).

C) exons 6-7: 5'-CTAGATGGAGGCAGCACTTTAAGACACTTTGG-3'
D) exons 8-9: 5'-AAGTAGGCCTAGGACCTGAGGCACTGGACG-3'
E) exons 6-9: 5'-TAAGTAGGCCTAGGACCTTTAAGACACTTTG-3'
F) exons 8-10: 5'-CAGGTTTAGGATATCCTGAGGCACTGGACG-3'
G) exon 13: 5'-CCTTCTCAGAGAGAAGTAAATTTACTTCTCTGTGG-3'
H) exons 12-14: 5'-CCACCATCTGCTTTCCCTTTCTTCTTCCTCC-3'
I) exons 11-14: 5'-CCACCATCTGCTTTCCTATGGCAATAACAATC-3'

### EXAMPLE 1

### Isolation of cDNA Clones:

Using the sequence of the 23 N-terminal amino acids previously obtained from the 66 and 56 kDa α and β chains of CD46 (Purcell et al. (1989) Immunol. Cell Biol. 67, 279-389), five oligonucleotide probes were synthesized (Table 1) using an Applied Biosystems 380A DNA Synthesizer (Applied Biosystems Inc.). Short oligonucleotides containing either the full component of redundant codons, or longer oligonucleotides selected to have the most likely codon using the algorithm of Lathe et al. (1985) J. Mol. Biol. 183, 1-12, were end-labelled with γ³² dATP using T4 polynucleotide kinase (Maxam and Gilbert (1977) Methods in Enzymology 65, 499-560) and unincorporated label removed with a Sepharose G-25 spun column.

A human placental cDNA library containing 5x10⁶ independent clones in the λgt11 vector (Clonetech) was plated at a density of 5x10⁴p.f.u. per 15 cm plate and plaque DNA transferred in triplicate and covalently linked to nylon membranes (Hybond-N, Amersham; Huynh et al. (1988) DNA Cloning I, A Practical Approach, Glover (Ed), Oxford pp 47-48. Filters were probed with three pools of oligonucleotides in hybridization buffer containing 5 x PE (1 x PE = 0.133m phosphate/1mM EDTA buffer, pH 6.9). 7% SDS, 0.5% BLOTTO (dried skim milk powder), 1 polyethylene glycol 2000 at 42°C and washed with 2xSSC (0.3M, Nacl, 0.03M Sodium Citrate pH 7.0), 0.1% SDS at 2°C below the lowest melting temperature of the oligonucleotides in the pool (Reed and Mann, 1985). Clones hybridizing with two or more of the pools were selected for further screening with both pools and individual oligonucleotides. After four rounds of screening, six clones (pm5.1, .2, .3, .6, .8 and .10), were isolated and phage DNA prepared by the DE52-Sepharose method (Benson and Taylor (1984) Bio Techniques, 2, 126-127). Inserts from cDNA clones were prepared by digesting with EcoRI and isolating insert bands by electrophoresis on an 0.8% agarose gel and subcloning into the pVZ plasmid vector for sequencing and other analyses.

DNA analysis. The nucleotide sequences of the CD46 cDNA clones were determined by the dideoxynucleotide chain termination method using the T4 polymerase, Sequenase II (Tabors and Richardson (1987) Proc. Natl. Acad. Sci. USA, 84, 4767-4777) (United States Biochemical Corporation) from double and single stranded templates of cDNA clone in the pVZ vector. Products were analyzed on denaturing acrylamide gels containing urea. The sequencing strategy included using internal oligonucleotide primers, and using clones containing cDNA inserts truncated by the Mung bean exonuclease III method (Sambrook et al. (1989) Molecular Cloning, A Laboratory Manual (2nd Ed), Cold Spring Harbour Laboratory) to sequence both strands of each clone. Sequences were collated and corrected and sequence searches performed with the aid of a VAX computer using the MELBSYS and DBQUERY suite of programs.

The nucleotide sequences of these clones were mostly identical, having a single open reading frame, however five of the six clones differed at the 3' end of the long open reading frame. Clone pm5.2 had a shorter 3' untranslated region than clone pm5.6 but was otherwise identical.

The complete nucleotide sequence of the pm5.1 clone, which had the longest contiguous coding sequence, is shown in Figure 1. The pm5.1 clone encodes a protein of 3.77 amino acids from the single long open reading frame, commencing from an initiation codon (ATG) at nucleotide position 33 or at position 102. The amino acid sequence commencing from the Cys at amino acid position 35 is identical to that obtained experimentally from purified CD46 (Purcell et al. (1990) Immunogenetics, 31, 21-28) and the sequence of the first 34 amino acids corresponds with that predicted for a typical leader peptide that is cleaved to generate the mature protein (von Heijne (1986) Nucleic Acids Res., 14, 4683-4690). The molecular weight calculated for the mature protein is 38,353 Da, which correlates well with earlier analyses of deglycosylated CD46 (Purcell et al. (1989) Immunol. Cell Biol., 67, 279-281) and biosynthetically labelled MCP precursor (Ballard et al. (1988) J. Immunol., 141, 3923-3929). There are three potential N-linked glycosylation sites (Figure 1) and multiple potential O-linked glycosylation sites, many of which exist in a region between amino acids 253 and 280. The addition of carbohydrate at these sites would account for the increased molecular weight of the mature protein. A stretch of 23 hydrophobic amino acids from position 295 to 317 are consistent with a membrane spanning domain (Kyte and Doolittle (1982) J. Mol. Biol., 157, 105-132). The rest of the open reading frame encodes a cytoplasmic tail of 26 mostly charged amino acids. Four regions of approximately 60 amino acids were identified at the NH₂-terminal end of the mature protein that have between 18-35% amino acid identity (Figure 2), and these each contain the consensus of the family of complement regulatory molecules and can therefore be considered as short consensus sequence repeat (SCR) units of this family of molecules (Reid et al. (1986) Immunol. Today, 7, 230-234).

The pm5.3, pm5.6 and pm5.10 clones each have segments of coding sequence deleted at regions 3' to the segment encoding the four SCR units of the prototype pm5.1 clone (Figure 1). The pm5.10 clone lacks 93bp of nucleotide sequence that encodes 16 of the 23 amino acids of the cytoplasmic tail and a portion of the 3' untranslated sequence of the pm5.1 clone (Figure 1, Figure 3). Since this 93bp fragment contains the termination codon ending the open reading frame of pm5.1, the new reading frame of pm5.10 includes a segment of sequence that forms the initial portion of the 3' untranslated sequence in pm5.1. Therefore, the CD46 molecule encoded by pm5.10 has a cytoplasmic tail of 33 amino acids, 23 of which differ from those encoded by pm5.1 (Figure 3). This new tail would be longer by 7 amino acids, but would also have a high proportion of charged amino acids. The pm5.10 clone is not full-length and lacks the sequence at the 5' end that would encode the leader peptide (Figure 4), however the remainder of the nucleotide sequence is identical to pm5.1. The nucleotide sequence of the pm5.10 clone perfectly matches within that of a MCP cDNA clone isolated independently, without a single nucleotide different (Lublin et al. (1988) J. Exp. Med., 168, 181-194).

The pm5.3 clone also lacks the 93bp fragment absent in pm5.10, but has an additional deletion of 42bp from a region encoding the second half (14 amino acids) of the Ser/Thr rich region (Figure 3, Figure 4) removing two potential O-linked carbohydrate addition sites and five kink forming Pro residues. Since loss of this segment of DNA does not change the reading frame the remainder of the CD46 protein encoded by this clone would have the same amino acid sequence as that encoded by pm5.10.

The pm5.6 clone also lacks the 93bp fragment absent in pm5.10, but has an additional deletion of 37bp from a region encoding the second half (12 amino acids) of the hydrophobic transmembrane domain resulting from the use of a cryptic splice acceptor sequence (TGTTGTCCCGTACAG) at the 3' and of this deleted segment (Figure 3, Figure 4). The absence of this fragment changes the reading frame and the CD46 protein encoded by the pm5.6 clone would have 34 different amino acids at the carboxyl-terminus compared to CD46 protein from the other clones. Subsequently, the resulting CD46 molecule would have hydrophilic and predominantly charged amino acids substituting for half of the transmembrane domain, and this may lead to secretion of this version of CD46 from the cell. The pm5.6 clone had a 3' untranslated region that was 465bp longer than the pm5.1 clone (Figure 1B). A second independent clone, pm5.2, had the same set of deletions as pm5.6, but was 286bp shorter than pm5.6 in the 3' untranslated region. Neither a poly(A) signal sequence (AATAAA) nor a poly(A) tail was found in the 3' untranslated sequences of any of the clones.

The sequence of the pm5.8 clone was identical to the pm5.1 clone in the portion encoding the NH² leader and four SCR regions, however the sequence after nucleotide 890 of pm5.1 was different to any sequence derived from all the other clones (Figure 3B) and results from reading through of the cDNA into an intron sequence after the fourth SCR (see below). If protein were encoded by this new sequence it would contain a stretch of 16 hydrophobic amino acids possibly serving as a membrane spanning sequence, although this is a little shorter than typically found (Kyte and Doolittle (1982) J. Mol. Biol., 157, 105-132). The putative cytoplasmic tail of this version of CD46 would also contain a high proportion of charged amino acids. The structure of the proteins derived from the 5 alternative versions of CD46 cDNA clones isolated from the human placental cDNA library are summarized in Figure 4.

### EXAMPLE 2

### Northern Blot Analysis of CD46 mRNA:

RNA was prepared from fresh human placental tissue using guanidinium isothiocyanate method (Chirgwin et al. (1979) Biochem., 18, 5294-5299) and poly(A)+ RNA isolated by chromatography on oligo (dT) cellulose (Aviv and Leder, (1972) Proc. Natl. Acad. Sci. USA, 69, 1408-1412). RNA was denatured and size fractionated on a 1% agarose gel in the presence of 0.8% formaldehyde and blotted onto nylon membranes using 0.1M NaOH transfer buffer. Filters were prehybridized with 4xPE defined earlier, 50% formamide, 7% SDS, 0.5% BLOTTO (skim milk powder), 1% polyethylene glycol at 42°C then hybridized with either pm5.1 cDNA insert labelled with 32Pα-ATP by nick transplantation, or with oligonucleotides complimentary to sequence spania the splice site or to or deleted segments of the cDNA clones (Table 2). Oligonucleotides were end-labelled using T4 polynucleotide kinase as before. Filters were washed in 0.1xSSC at 60°C for the cDNA probes or 1xSSC at 1°C below the Tdmin for the oligonucleotides.

The size and number of CD46 mRNA species was determined in samples of spleen from human and gibbonape cell lines (Fig. 5). Each of the samples tested had a dominant band at 3.2kb and the spleen infiltrated with hairy cell leukaemia (Fig. 5c) had two other fainter bands of 4.8 and 7.7kb. Using densitometric scanning equivalent levels of the 3.2kb CD46 mRNA were observed in spleen from patients with non-Hodgkins lymphoma and polycythemia vera and in the human cell-lines PEER and U937 and in the gibbon ape cell line 144-MLA. The 144-MLA line is chronically infected with the gibbon ape leukaemia virus which encodes a virion surface glycoprotein (SU7O) that bears a cross-reactive antigenic epitope of CD46 recognised by the E4.3 mAb (Purcell et al. (1989) Leukocyte Typing IV White Cell Differentiation Antigens, pp 653-655, Oxford University Press). The E4.3 mAb to CD46 recognised thirty fold more protein at the surface of 144-MLA cells than other human cell lines, however the equivalent levels of CD46 mRNA supports the earlier finding that the E4.3 mAb predominantly reacts with the gibbon ape leukaemia virus surface gp70 molecules on the 144-MLA cell. The spleen sample infiltrated with hairy cell leukaemia showed a ten fold greater level of the 3.2kb CD46 mRNA than the other spleen samples and the histopathologically normal spleen from a trauma patient had twenty fold less CD46 mRNA than the spleen samples infiltrated by neoplastic cells. This again showed that malignant tissue samples and cell-lines contain ten to twenty fold more CD46 than the corresponding non-malignant cell and shows that this is due to elevated transcription of CD46 RNA. Size variations of around 100bp in the 3.2 kb band, as predicted from the sequence of the clones, were beyond the resolution capability of these gels. To verify the existence of, and examine the relative proportion of, the alternatively spliced CD46 RNA species predicted from the clones, a series of antisense oligonucleotides were constructed that would differentially hybridize with alternately spliced CD46 mRNA (Table 2). These were used to probe Northern blots of poly(A)⁺ RNA from human placenta (Fig. 6). When a CD46 cDNA insert from the pm5.1 clone was used as a probe three bands were noted, a broad band at 3.2kb as with the other human cells, a band of 1.9kb and a fainter band of 5.2kb. The 1.9kb band probably relates to the use of cryptic polyadenylation signals (AATATA or AATGAA) found at positions 1579 and 1592 of the pm5.1 clone. These sequences were apparently used by the clone of MCP isolated by Lublin et al. (1988) J. Exp. Med., 168, 181-194. Hybridization with the On63 oligonucleotide, specific for RNA lacking the 93bp segment of the pm5.1 clone between positions 1115 and 1208 (i.e., corresponding to the pm5.3, pm5.6 and pm5.10 clones), yielded a faint band of 3.2kb (arrowed in Fig. 6b) showing that RNA lacking the 93bp segments is a genuine component of the pool of CD46 mRNA. Hybridization with the On46 oligonucleotide, specific for RNA containing the 93bp segment (pm5.1) yielded a predominant 3.2 kb CD46 band (Figure 6c). Hybridization with the On35 oligonucleotide, which is specific for RNA containing pm5.3 type of deletion of 42bp, also yielded the 3.2kb CD46 RNA band verifying that a subpool of CD46 RNA contains this deletion (Fig. 6d). Hybridization of the On44 oligonucleotide, which is specific for RNA retaining the 42 bp deletion yielded a very faint 3.2kb CD46 RNA band showing that a subpool of CD46 RNA retains this deleted segment (Fig. 6e). Hybridization with the On68 oligonucleotide, which is specific for the new (intron) sequence of the pm5.8 clone, did not result in the detection of any RNA bands (Fig. 6f). The results show that the pm5.1, pm5.3, pm5.6 and pm5.10 cDNA clones of CD46 isolated from the placental cDNA library result from the presence of corresponding alternative RNA species for CD46 and are not a result of rearrangements during the construction of the library or other manipulations.

### EXAMPLE 3

### Analysis of CD46 mRNA by Polymerase Chain Reaction (PCR):

Placental poly(A)⁺ RNA and total RNA was treated with RNase-free DNase and used as a template for the synthesis of cDNA with 100U Molony murine leukemia virus reverse transcriptase. Reactions of 5µg poly(A)⁺ RNA or 50µg total RNA; 12.5µM (each) dNTPs, 0.25µM On24, and oligo (dT) 16-20 mer or random hexamers as primers were incubated at 37°C for 1 hour, then DNA was amplified by Polymerase Chain Reaction (PCR) in 0.5mM MgCl₂, 10mM Tris HCl (pH 8.3), 50mM KCl, 200mM (each) dNTPs, 4UTaq DNA polymerase (Amersham). Pairs of oligonucleotides were added to a final concentration of 1µM and template was added at 2µl, 1µg, or 1ng per 50µl reaction for first strand cDNA, genomic DNA and cDNA clones respectively. To label the product the dCTP concentration was reduced to 100mM and 0.5µl α³²P-dCTP was added. After 3 min. at 95°C reactions were subjected to 30 cycles of denaturation (93°C, 1 min.) annealing (see Table 2, 2 min.), and elongation, (72°C, 1 min). Unlabelled PCR products were size fractionated on a 1.5% agarose gel transferred to Hybond N⁺ in 0.5M NaOH, 1.5M NaCl for probing with oligonucleotides.

Radiolabelled PCR samples were analyzed by electrophoresis on 6% acrylamide gels containing urea with sequencing reactions as size markers and autoradiographed at -70°C on XAR-5 or XRP film.

To overcome the weak hybridization signals obtained in Northern blots using the type specific oligonucleotide probes, placental poly(A)+ cytoplasmic RNA was converted to cDNA with reverse transcriptase by priming with On24 or with oligo(dT) and alternate cDNAs for CD46 amplified by polymerase chain reaction (PCR) using CD46 primers On57 and On24 that hybridize outside the region of alternative splicing. Electrophoresis of labelled amplification products from the different CD46 clones and from placental cDNA on 6% acrylamide gels revealed a number of different sized bands (Fig. 7A), including bands corresponding in size to those amplified from the pm5.1, pm5.3, pm5.6 and pm5.10 clones on (391, 256, 261 and 298bp respectively). Several additional bands (219, 247, 289, 304 and 349bp) were observed from placental cDNA samples primed with both On24 and oligo(dT). While several of these extra bands differ from the 391bp pm5.1 - type band by sizes that may correspond to the deletion of different sets of the 37, 42 and 93bp segments (eg. 349 = 391-42, or 219 = 391-37-42-93) or of the 27 and 75bp segments between the 42, 37 and 93bp regions (eg. 289 = 391-75-27, or 249 = 391-42-75-27) the structure of these and their significance requires further investigation. A doublet of 415 and 419bp which is larger than the PCR products from any of the clones was also produced from cDNA primed with On24 but not with oligo(dT). The RNA molecules giving rise to these bands either lack a poly(A) tail or exist in the 5.2kb poly (A)+RNA pool which would be reverse transcribed to the region of alternative splicing at low efficiency and may not be functionally important. None of the bands in the cDNA samples arose due to amplification of contaminating clones or genomic DNA because no bands were obtained after PCR from various controls including reaction mixes lacking template DNA, cDNA reaction mixes lacking reverse transcriptase (hence containing only RNA), an aliquot of reverse transcriptase enzyme mix used in cDNA synthesis and genomic DNA. To confirm the identity of the different bands amplified from cDNA, unlabelled PCR products were transferred to nylon filters after agarose gel electrophoresis and probed with the type - specific oligonucleotides (Fig. 7B). Probing with On63 for the 93bp deletion of pm5.3, pm5.6 and pm5.10 (Panel 2) revealed a smear of bands from the position of the pm5.10 band to below the pm5.3 and pm5.6 bands. Probing within the 93bp sequence with On46 showed a smear of several bands around the size of the pm5.1 band (Panel 3). Probing with On44 for the 42bp sequence deleted in pm5.3 (Panel 4) detected several bands corresponding in size to the bands from the pm5.1, pm5.6 and pm5.10 clones. The On35 probe spanning the 42bp deletion (Panel 5) also detected several bands including one of similar size to the pm5.3 band. The On45 probe which lies within the 37bp deletion (Panel 6,) detected several bands corresponding to pm5.1, pm5.3 and pm5.10. Each of these oligonucleotide probes confirmed the existence of RNA molecules in placenta that have the deletions identified in the CD46 cDNA clones however these probes detected with more PCR products amplified from cDNA than accounted for by amplification from the clones. This shows that several of the extra bands seen in Figure 7A result from different combinations of the deleted segments contained in the clones.

Because the On36 oligonucleotide which spans the site of the 37bp deletion failed to discriminate pm5.6 from the other CD46 clones when used as a probe in hybridization studies, we used On35 as a primer to amplify placental cDNA prepared with oligo(dT) priming by PCR (Fig. 8, panel 1). A product of 756bp was amplified from the pm5.6 clone and from placental cDNA but not from controls. Two larger bands of unknown identity were also obtained from the PCR with placental cDNA. These results show that the 37bp deletion of the pm5.6 and pm5.2 clones is due to RNA with this structure and not due to cloning artifact.
Further, PCR amplification reactions were performed on cDNA from cytoplasmic RNA and genomic DNA using primers at either side of the boundaries of the deleted segments and the regions separating these to locate the intron/exon junctions (Table 3). After PCR across their boundaries, the 42 and 93 bp deleted segments, and the 45bp region between the site of the pm5.8 alteration and before the 42bp deletion of pm5.3, were each found to be separate exons yielding larger or no bands with genomic DNA template compared with cDNA template. Introns were located within the 75bp region between the 42 and 37bp deletions and the 5' boundary of the 37bp deletion site. By contrast, no intron was identified at the 3' boundary of the 37bp deletion, but rather at the boundary of the adjacent 27bp region and the 93bp segment. This suggests that the 37 and 27bp regions are combined to form a single exon. The existence of an intron at the position where the pm5.8 sequence differs from the other clones and the presence of a consensus splice donor sequence (AAGGTACAA) at the site of sequence divergence led us to examine if this clone resulted from the failure to splice this intron. Genomic DNA from placenta and cDNA made by priming poly (A)+ cytoplasmic RNA with random hexamers were used as template in PCR using On85 and On83 to amplify across the region of sequence difference (Fig. 8, Panel 2). A band of 300bp was produced from the pm5.8 control and from genomic DNA, but not from cDNA or other controls, showing the different sequence in pm5.8 is due to the failure of removal of this intron.

The technique of PCR has been applied to examine the alternative RNAs from different tissues using On85 prepared to a unique stretch of nucleotide sequence in the fourth SCR (Table 2) and On24 in the 3' intranslated region beyond the alternatively spliced region. These were used in PCR using single stranded cDNA template reverse transcribed from RNA from many cell types including several placenta, peripheral blood leukocytes, leukaemia cells from patients with several different malignancies, spleen from trauma, leukaemia and lymphoma patients and cell lines derived from lymphoid and solid tumours. This analysis has shown several interesting points; i) the serine/threonine rich region of CD46 is composed of three separate exons, the third of these was deleted from the pm5.3 clone of the CD46 the second is the 45bp region between the sites of the pm5.3 and pm5.8 deletions and the first is another 45bp segment that is not represented in any of our placental cDNA clones; ii) the first of the three Ser/Thr region exons is rarely included in spliced RNA in placenta; iii) the third Ser/Thr region exon is rarely included in spliced RNA from several cell-lines, but is commonly spliced from RNA from placentae; iv) the second Ser/Thr region exon is alternatively spliced in the RNA of most tissues examined to date; v) one 93bp exon containing an inframe termination condon is alternately spliced to yield two different cytoplasmic tails corresponding to the two cytoplasmic tails found in our pm5.1 and pm5.10 clones, all cell types examined to date have alternately spliced this exon in the pool of RNAs encoding several CD46 isotypes; vi) a third type of cytoplasmic tail used in the pm5.6 clone arises is some cell types due to the use of a cryptic splice acceptor sequence within the second of two exons encoding the transmembrance domain, generating a potentially secreted from of CD46. The PCR technique has been useful in mapping the intron/exon boundaries and identifying that different spliced RNA species exist in different cell types, however most CD46 RNA splice variants are common to all cell types.

Synthetic peptides corresponding to the alternative carboxyl-terminal sequences were used to generate antiserum specific for CD46 isoforms. These CD46 isotype - specific antisera were used in conjunction with our E4.3 monodonal antibody which reacts with all isotypes of CD46 in an enzyme linked immunosorbant assay that specifically detects CD46 variants containing the alternative fragments of nucleic acid identified in the alternative cDNA clones (pm5.1, 5.3, 5.6 and 5.8).

As will be apparent from the foregoing, the presence of spliced RNAs encoding CD46 was totally unexpected.

The above described Examples and conclusions were consolidated to characterize the molecular events leading to the isoform heterogeneity and polymorphic expression of CD46. In particular CD46 and RNA from different tissues was examined. Fourteen alternatively spliced RNA transcripts (several of which correspond to pm.5 series clones described above) were found to be differently expressed, explaining the heterogenous nature of CD46 in tissue.

In the following Examples materials and methods are as follows:
**Tissues.** Heparinized blood from healthy donors or leukemic patients with chronic or acute leukemia was fractionated by centrifugation through Ficoll-Paque (Pharmacia, Uppsala, Sweden) or Mono-Poly Resolving Medium (ICN, Irvine, CA) according to manufacturers instructions, to obtain lymphocytes and granulocytes. The EBV-transformed B cell line was produced in the Research Center for Cancer and Transplantation, The University of Melbourne. Full term placentae, a resected colon tumor and adjacent normal colon were separately homogenized for RNA extraction or teased into single cell suspensions for protein extraction. Semen samples obtained from healthy volunteers were used unfractionated for RNA extraction; spermatozoa were isolated from protein analysis by liquefying for 30 min at room temperature, centrifuging for 10 min at 700 g and washing (x4) with phosphate buffered saline.
**Western blot.** Cell lysates were prepared at 5 x 10⁷ cells/ml (or 5 x 10⁸ spermatozoa/ml) in 0.5% Nonidet-P40 in 10 mM Tris, 0.15 NaCl, pH 7.4 containing 1 mM EDTA and 1 mM phenylmethylsulfonyl fluoride. Cell lysates were acid/base dissociated by the techniques of Swack et al. (1987) Biotechniques, 5, 564-571 and 30 µg aliquots of protein (measured using the Biorad protein assay) were separated by SDS-PAGE under non-reducing conditions (see Laemmli (1970) Nature 227, 680-685). Proteins were electroblotted onto Immobilon P membrane (Millipore, Bedford, MA) and the remaining protein-binding sites were blocked with 2% casein. The CD46 isoforms were detected by the anti-CD46 monoclonal antibody, E4.3 described in Sparrow et al. (1983) Hum. Immunol. 7, 1-15, followed by incubation with horseradish peroxidase-conjugated antimouse IgG reagent (Dakopatts, Denmark) and visualisation with a cobalt-enhanced diaminobenzidine substrate.
**cDNA synthesis and PCR amplification.** Total RNA was prepared using the guanidinium isothiocyanate method (Chirgwin et al. (1979) Biochem. 18, 5294-5299). First strand cDNA was synthesized from 10 µg RNA in 25 µl using 200 U Murine Leukemia Virus reverse transcriptase (BRL, Gaithersburg, MD), 0.25 mM dATP, dGTP, dTTP, dCTP (each) and 0.5 µg random hexamer oligonucleotides. PCR amplification was performed with 1U 'Replinase' (NEN, Boston, MA), 5µl first strand cDNA, 0.2 mM dATP, dGTP, dTTP, dCTP (each) and 1 µM each of primers P1 and P2 in 50 mM Tris-HCl (pH9.0), 20 mM (NH₄)₂SO₄, 1.5 mM MgCl₂, with or without 2.5 µCi α[³²P]dCTP. After 2 min at 95°C, 1 min), annealing (57°C, 2 min), and elongation (72°C, 2 min). Stringent precautions were taken to avoid cross-contamination between the RNA and cDNA samples, including their preparation in a laboratory in which CD46 clones and PCR products were never handled. Controls, including no cDNA and individual cDNA clones are templates, were included in each PCR to confirm that cross-contamination did not occur.
**Analysis of PCR products.** The amplified PCR product was extracted with chloroform, and 5 µl aliquots (5% of total product) were electrophoresed on 6% denaturing polyacrylamide gels and autoradiographed. For nucleotide sequencing, bands were excised and eluted in 0.5 M NH₄OAc, 10 mM MgOAc, 1 mM EDTA, 0.1% SDS for 16 hrs at 4°C, then precipitated with ethanol. 25% of each sample was reamplified with the same primers, extracted with chloroform and purified using Elutip-d columns (Schleicher and Schuell, Dassel, Germany). The sample was denatured with 0.2 M NaOH, neutralised, precipitated with ethanol and sequenced using [³²P]-labelled primers and T7 DNA polymerase (Amersham, Poole, UK). To assess the expression of each splice variant in different tissues, cDNA was amplified in the absence of α[³²P]dCTP and electrophoresed on 6% denaturing polyacrylamide gels, electroblotted onto nylon membranes in 45 mM Tris-borate, 2 mM EDTA, fixed with 0.25 M NaOH, 1.5 M NaCl, and probed with oligonucleotides specific for alternatively spliced exons or groups of exons (see Figure 12 for location of exons).

### EXAMPLE 4

### Tissue Specific Expression of CD46 Protein Isoforms:

Western blot analysis of the different Mᵣ forms of CD46 protein in 16 tissues demonstrated several important features of CD46 protein heterogeneity (Figure 9). **1**) A 76 kDa isoform (termed γ) was observed in EBV-transformed B cells (lane 5) and two of the three leukemic samples (lanes 8, 10). **2**) Lymphocytes from healthy donors and cells from leukemic patients contained CD46 isoforms that resolves into two major clusters of bands at 66 and 56 kDa (termed α and β respectively, previously called upper and lower), whose relative abundance varied according to an autosomal codominant polymorphism (9). The three phenotypes are: α-predominant (lanes 1 and 8); α and β in equal proportions (lanes 2 and 9); and β-predominant (lanes 3 and 10). **3**) Some placentae expressed an isoform of 63 kDa (termed ξ, lanes 14-16), not seen in the other tissues, in addition to the α and β isoforms (lanes 13-16). **4**) Spermatozoa expressed a unique 35 kDa form of CD46 (termed 6, lanes 6 and 7) not found in the other tissues examined, they did not express the α and β isoforms. It was also noted that granulocytes showed isoforms up to 78 kDa (termed ζ) together with the typical α and β isoforms (lane 4). Both normal colon and colon carcinoma tissue from a single donor expressed predominantly α CD46 isoforms with no difference between malignant and normal tissue (lanes 11 and 12). Thus, there are two types of polymorphism apparent here:
a) allelic differences in the proportions of α and β isoforms; and b) tissue differences in expression of the α, β, γ, δ, ξ and ζ isoforms.

### EXAMPLE 5

### Structure of CD46 RNA Transcripts:

To investigate alternative splicing of CD46 mRNA, we first identified all alternatively spliced RNAs from the tissues described above, a number of placentae and lymphocyte samples, and from hemopoietic cell lines. RNA from these tissues was converted to cDNA and amplified, and all new splice variants identified were sequenced. No alternative splicing of RNA in the SCR region was observed (data not shown), thus excluding exons 1-6 (Figures 9, 12) from contributing to protein heterogeneity. Amplification and sequencing between the fourth SCR and the 3' untranslated region (primers P1 and P2, Figure 10A), however, identified 14 differently spliced RNAs. Sequencing indicated that most transcripts were derived from the alternative splicing of exons 8, 9, 12 and 13 shown in Figure 10B, Figure 12, but other RNAs contained new sequence immediately downstream of the SCR region (Figure 10C). This sequence was designated exon 7 (STP A) because of its position in CD46 mRNA and its derived amino acid sequence, and completes the description of the exons identified in genomic clones for CD46. The nucleotide sequence of exon 7 was homologous to the sequence of the two other STP-encoding exons, as there was 69% identity with exon 8, and 62% identity with exon 9. The splicing of exons 7, 8 and 9 did not change the reading frame but effected the number of potential O-glycosylation sites as this region is rich in Ser and Thr residues. Exon 10 is of unknown UK significance and its boundary with exon 11 not yet comprehensively delimited. Exon 13 encoded a cytoplasmic tail (Figure 10, D3) or all (Figure 10, D4) of exon 12 changed the reading frame, giving rise to a third cytoplasmic tail and removing part of the transmembrane region. Thus, alternative splicing of five exons (7, 8, 9, 12 and 13) results in CD46 RNAs encoding protein isoforms with three different cytoplasmic tails and with differences in the length of both the hydrophobic transmembrane region and the STP-rich region of the glycoprotein.

### EXAMPLE 6

### Tissue Distribution of Alternatively Spliced CD46 RNAs:

To determine the relative expression of the 14 RNA transcripts in different tissues, RNA from each tissue examined in Figure 9 was converted to cDNA and amplified by PCR using primers P1 and P2, spanning the alternatively spliced region. Amplification of CD46 cDNA clones (Figure 11A) and multiple analysis of RNA samples showed that, although amplification did not allow the estimation of the total amount of CD46 in the sample, it is provided an accurate and reproducible estimation of the relative proportions of the different CD46 RNA variants. We note that no additional splice patterns were produced by this procedure, indicating that homologous recombination during amplification did not occur in these experiments. When α[³²P]dCTP was incorporated into the PCR products, the 14 transcripts migrated as only 6 bands after electrophoresis (Figure 11B) due to the similar size of some of the exons. To identify the distribution of each transcript, unlabelled PCR products were electrophoresed, transferred onto nylon filters and probed with oligonucleotides specific for each splice variant (see Figure 12 for location of probes). Fifteen oligonucleotides were used to determine the distribution of the 14 RNAs (representative gels in Figures 11C-I).

Five observations merit particular attention:
**1**) CD46 RNAs containing exon 7 (Figure 11C) were preferentially expressed in EBV-transformed B cells (lane 5) and in two leukemic samples (lanes 8 and 10). RNAs containing exon 7 were also expressed in placentae, although to a lesser extent (lanes 13-16). **2**) The ratio of the quantity of transcripts containing exon 8, to transcripts in which exon 8 have been deleted, was equivalent to the ratio of α and β isoforms detected on western blots in every tissue excepting sperm (Figures 9, 11D, 11E). This demonstrated that the α isoforms were derived from RNAs in which exon 8 had been inserted, and the β isoforms from RNAs in which exon 8 had been deleted. **3**) Probing for the deletion of exon 9 demonstrated that RNA transcripts of this type occurred in 3 or the 4 placentae examined (Figure 11F, lanes 14-16). **4**) A transcript in which exons 12 and 13 were deleted occurred only in semen (Figure 11I, lanes 6 and 7). **5**) RNA transcripts both with and without exon 13 were present in every tissue and the ratio of these two groups of transcripts was similar in all tissues (Figure 11G, 11H), Thus, each splice variant displayed a different distribution of expression, with tissue specific and allelic variations in splicing and with some RNAs expressed in every tissue.

### EXAMPLE 7

The sequences of the different CD46 variants were subjected to a computer analysis and homologies with sequences of known function were determined using "Prosearch": Lee F. Kolakowski's search of protein sequences against Amos Bairoch's PROSITE database. Sequences corresponding to known phosphorylation sites and nuclear localisation sites were identified.
(a) A sequence associated with cyclic AMP-dependent phosphorylation (KKGT) was identified in the "CYT1" cytoplasmic tail corresponding to the pm5.1 clone. This tail also contained the consensus sequence for Protein kinase C dependent phosphorylation (THR).
(b) Sequences associated with casein kinase 2 phosphorylation were identified in both the CYT 1 (pm5.1) and CYT 2 (pm5.3, pm5.10) cytoplasmic tails (THRE and TPAE respectively).
(c) The sequence KKKGK of the CYT 2 cytoplasmic tail has been identified as a nuclear localisation signal.

These results are suggestive of a signal role possibly phosphorylation based for portions of the CD46 molecule remote from the SCR domains active in the complement cascade. The activity status of the SCR domains could trigger this signal role through, for instance, conformation changes in the molecule. The signal is conceivably implicated in feedback or cascade enhancement roles.

The drawings herewith are incorporated in this specification.

## Claims

1. A recombinant nucleic acid encoding:
(a) a CD46 isoform defined by cDNA clone pm5.1, as shown in Figure 4 in conjunction with Figure 1A;
(b) a CD46 isoform defined by cDNA clone pm5.3, as shown in Figure 4 in conjunction with Figure 1A;
(c) a CD46 isoform defined by cDNA clone pm5.6, as shown in Figure 4 in conjunction with Figure 1A; or
(d) a CD46 isoform defined by cDNA clone pm5.8, as shown in Figure 4 in conjunction with Figure 1A.

2. A probe comprising a nucleic acid according to Claim 1 and a detectable marker linked thereto.

3. A eukaryotic or prokaryotic transfer or expression vector comprising a nucleic acid sequence according to Claim 1 and a promoter sequence therefor.

4. A host cell compatible with and incorporating a vector according to Claim 3, whereby the host cell is capable of expressing the CD46 isoform.

5. A host cell having a nucleic acid according to Claim 1 operatively integrated within its genome, whereby the cell is capable of expressing the CD46 isoform.

6. A CD46 isoform produced by a cell according to Claim 4 or 5.

7. A CD46 isoform according to Claim 6 in soluble form.

8. A pharmaceutical composition comprising a CD46 isoform according to Claim 6 or 7 in conjunction with a pharmaceutically acceptable carrier or diluent.

9. The use of a CD46 isoform according to Claim 6 or 7 in the manufacture of a medicament or diagnostic reagent.

10. The use of a nucleic acid according to Claim 1 in the manufacture of a probe or antisense therapeutic agent.

11. A CD46 isoform according to Claim 6 or 7 for use in a method of inhibiting complement activation or inflammation mediated immunoresponse in a mammal.

12. An antisense oligonucleotide which is complementary to at least a substantial portion of exon 7 of a recombinant nucleic according to Claim 1 for use in a method of inhibiting leukaemic cells, the method comprising administering antisense oligonucleotide to an animal, the antisense oligonucleotide serving to diminish the expression of CD46 by leukaemic cells thereby rendering the cells vulnerable to complement.

13. A nucleic acid probe according to Claim 2 for use in a method of diagnosing the likelihood of spontaneous abortion in a mammal, the method comprising applying the probe to tissue or fluid of the mammal or its foetus.

14. A CD46 isoform according to Claim 6 or 7 for use in a method of maintaining pregnancy in an habitually aborting mammal, the method comprising the administration of the CD46 isoform to the mammal to thereby mop up complement.

15. A CD46 isoform according to Claim 6 or 7 for use in a method of preventing inhibition of mammalian sperm function, the method comprising the administration of an effective amount of the CD46 isoform to the sperm or its environment.

## Patentansprüche

1. Rekombinante Nukleinsäure, die folgendes kodiert:
(a) eine CD46-Isoform, die von cDNA-Klon pm5.1 definiert ist, wie in Figur 4 in Verbindung mit Figur 1A gezeigt ist;
(b) eine CD46-Isoform, die von cDNA-Klon pm5.3 definiert ist, wie in Figur 4 in Verbindung mit Figur 1A gezeigt ist;
(c) eine CD46-Isoform, die von cDNA-Klon pm5.6 definiert ist, wie in Figur 4 in Verbindung mit Figur 1A gezeigt ist;
(d) eine CD46-Isoform, die von cDNA-Klon pm5.8 definiert ist, wie in Figur 4 in Verbindung mit Figur 1A gezeigt ist;

2. Sonde, die eine Nukleinsäure nach Anspruch 1 und einen damit verknüpften auffindbaren Markierer aufweist.

3. Eukaryotischer oder prokaryotischer Übertragungs- oder Ausdrucksvektor, der eine Nukleinsäuresequenz nach Anspruch 1 aufweist und eine Promotorsequenz dafür.

4. Wirtszelle, die mit einem Vektor nach Anspruch 3 verträglich ist und ihn umfasst, wobei die Wirtszelle die CD46-Isoform ausdrücken kann.

5. Wirtszelle mit einer Nukleinsäure nach Anspruch 1, die wirkend in ihrem Genom integriert ist, wobei die Zelle die CD46-Isoform ausdrücken kann.

6. CD46-Isoform hergestellt von einer Zelle nach Anspruch 4 oder 5.

7. CD46-Isoform nach Anspruch 6 in löslicher Form.

8. Pharmazeutische Zusammensetzung, die eine CD46-Isoform nach Anspruch 6 oder 7 aufweist, in Verbindung mit einem pharmazeutisch annehmbaren Träger oder Verdünnungsmittel.

9. Benutzung einer CD46-Isoform nach Anspruch 6 oder 7 bei der Herstellung eines Medikaments oder diagnostischen Reagenz.

10. Benutzung einer Nukleinsäure nach Anspruch 1 bei der Herstellung einer Sonde oder einem therapeutischem Antisensemittel.

11. CD46-Isoform nach Anspruch 6 oder 7 zur Benutzung in einem Verfahren zum Inhibieren von Ergänzungsaktivation oder -entzündung vermittelte Immunreaktion in einem Säugetier.

12. Antisense-Oligonukleotid, das zu wenigstens einem wesentlichen Teil von Exon 7 eines rekombinanten Nukleins nach Anspruch 1 komplementär ist, zur Benutzung in einem Verfahren zum Inhibieren von leukämischen Zellen, wobei das Verfahren aufweist, Antisense-Oligonukleotid an ein Tier zu verabreichen, wobei das Antisense-Oligonukleotid dazu dient, den Ausdruck von CD46 von leukämischen Zellen zu verringern, dabei die Zellen der Ergänzung gegenüber verletzlich machen.

13. Nukleinsäurensonde nach Anspruch 2 zur Benutzung bei einem Verfahren zum Diagnostizieren der Wahrscheinlichkeit der spontanen Abtreibung in einem Säugetier, wobei das Verfahren aufweist, die Sonde auf Gewebe oder Flüssigkeit des Tiers oder seinen Fötus anzuwenden.

14. CD46-Isoform nach Anspruch 6 oder 7 zur Benutzung in einem Verfahren zur Erhaltung von Schwangerschaft in einem ständig abtreibenden Säugetier, wobei das Verfahren die Verabreichung der CD46-Isoform an das Säugetier aufweist, um dabei die Ergänzung zu vernichten.

15. CD46-Isoform nach Anspruch 6 oder 7 zur Benutzung bei einem Verfahren zum Verhindern der Inhibierung von Säugetiersamenfunktion, wobei das Verfahren die Verabreichung einer effektiven Menge der CD46-Isoform an den Samen oder seine Umgebung aufweist.

## Revendications

1. Acide nucléique recombinant codifiant:
(a) un isoforme CD46 défini par le clone ADNc pm5.1, tel qu'indiqué en Figure 4 en association avec la figure 1A;
(b) un isoforme CD46 défini par le clone ADNc pm5.3, tel qu'indiqué en Figure 4 en association avec la Figure 1A;
(c) un isoforme CD46 défini par le clone ADNc pm5.6, tel qu'indiqué en Figure 4 en association avec la Figure 1A; ou
(d) un isoforme CD46 défini par le clone ADNc pm5.8, tel qu'indiqué en Figure 4 en association avec la Figure 1A.

2. Une sonde comportant un acide nucléique selon la revendication 1 et un marqueur détectable qui lui est associé.

3. Un vecteur de transfert ou d'expression eucaryotique ou procaryotique comportant une séquence d'acide nucléique selon la revendication 1 et une séquence promotrice en conséquence.

4. Une cellule hôte compatible avec et incorporant un vecteur suivant la revendication 3, selon laquelle la cellule-hôte permet d'exprimer l'ísoforme CD46.

5. Une cellule hôte comportant un acide nucléique selon la revendication 1 intégrée en fonctionnement dans son génome, selon laquelle la cellule permet d'exprimer l'isoforme CD46.

6. Un isoforme CD46 produit par une cellule selon la revendication 4 ou 5.

7. Un isoforme CD46 de forme soluble selon la revendication 6.

8. Un composé pharmaceutique comportant l'isoforme CD46 selon la revendication 6 ou 7 en association avec un porteur ou diluant pharmaceutique admissible.

9. L'exploitation d'un isoforme CD46 selon la revendication 6 ou 7 lors de l'élaboration d'un diagnostique de médicament ou d'agent réactif.

10. L'exploitation d'un isoforme CD46 selon la revendication 1 dans l'élaboration d'une sonde ou d'un agent thérapeutique antisensibilité.

11. L'ísoforme CD46 selon la revendication 6 ou 7 servant au minimum à l'inhibition de l'activation complémentaire ou de l'immuno-réponse interpolée par inflammation auprès d'un mammifère.

12. Un oligonucléotide d'antisensibilité qui est au minimum complémentaire à l'effet d'une portion importante d'exon 7 d'un recombinant nucléique selon la revendication 1 servant à un mode ínhibiteur de cellules leucémiques, le mode comportant le traitement d'un animal par oligonucléotide antisensibilité administré, ledit oligonucléotide antisensibilité servant à réduire l'expression du CD46 par des cellules leucémiques, celles-ci étant ainsi rendues vulnérables à la complémentarité.

13. La sonde d'acide nucléique selon la revendication 2 servant de méthode de diagnostique de probabilité d'avortement spontané d'un mammifère, la méthode prévoyant l'application par la sonde au tissu ou fluide ou au foetus du mammifère.

14. L'isoforme CD46 à la revendication 6 ou 7 servant de méthode de maintien de grossesse d'un mammifère en avortement habituel, la méthode prévoyant l'isoforme CD46 administré au mammifère pour en éponger le complément.

15. L'isoforme CD46 selon la revendication 6 ou 7 servant de méthode pour enrayer l'inhibition de la fonction de sperme animal, la méthode comportant l'administration d'un volume valable d'isoforme CD46 au sperme ou à son environnement.
